(19) Europäisches Patentamt
European Patent Office
Office européen des brevets

(11) Publication number: **0 201 203**
**A2**

(12) # EUROPEAN PATENT APPLICATION

(21) Application number: **86302523.5**

(22) Date of filing: **04.04.86**

(51) Int. Cl.⁴: **A01N 37/44**, A01N 47/02, C07C 101/453, C07C 121/78

(30) Priority: **04.04.85 GB 8508824**
**08.01.86 GB 8600372**
**31.01.86 GB 8602413**

(43) Date of publication of application:
**17.12.86 Bulletin 86/46**

(84) Designated Contracting States:
**AT BE CH DE FR GB IT LI LU NL SE**

(71) Applicant: **MAY & BAKER LIMITED**

**Dagenham Essex RM10 7XS(GB)**

(72) Inventor: **Feldwick, Graham Alan c/o May & Baker Limited**

**Dagenham Essex RM10 7XS(GB)**
Inventor: **Hatton, Leslie Roy c/o May & Baker Limited**

**Dagenham Essex RM10 7XS(GB)**

(74) Representative: **Bentham, Stephen et al**
**J.A. Kemp & Co. 14 South Square Gray's Inn London WC1R 5EU(GB)**

(54) **Method of treatment of plants with aniline derivatives.**

(57) A method of regulating plant growth at a locus which comprises applying to the locus an effective amount of a compound of the general formula:

$R^3NHC(COOR^1) = CH(COOR^2)$ (I)

wherein $R^1$ and $R^2$, which are the same, each represent a straight-or branched-chain alkyl group containing from 1 to 6 carbon atoms and $R^3$ represents a phenyl group bearing substituents $R^4$ and $(R^5)_n$;

wherein $R^4$ represents a cyano group, a straight-or branched-chain alkyl group containing from 1 to 4 carbon atoms which is fully substituted by halogen atoms, which may be the same or different, in the meta or para position; a hydroxy group or a fluorine or iodine atom in the meta position; a chlorine or bromine atom or a nitro group or straight-or branched-chain alkoxy or alkylthio group containing from 1 to 4 carbon atoms which is fully substituted by halogen atoms, which may be the same or different, in the para position and $R^5$ represents a hydrogen atom and n is 1;

or $R^4$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halogen atoms, which may be the same or different, and $R^5$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halogen atoms, which may be the same or different, and n is 1 or 2;



Rank Xerox

it being understood that:

a) when n is 1, $R^4$ and $R^5$ do not both represent unsubstituted alkyl groups, hydroxy groups or nitro groups and are different when in the 2,3-or 2,5-positions,

b) when n is 2, one of the substituents represented by $R^4$ and $R^5$ is different from the others and does not represent an unsubstituted alkyl group, the positions of the various substituents on the phenyl ring referred to above being with respect to the nitrogen atom to which the phenyl ring is attached, is disclosed.

Those compounds of the general formula depicted in Figure I which are new and their processes of preparation are further features of the present invention.

"METHOD OF TREATMENT OF PLANTS WITH ANILINE DERIVATIVES"

The present invention relates to a method for regulating plant growth by applying certain aniline derivatives to the plants and/or their environment and to compositions for regulating plant growth containing such compounds as an active ingredient.

In the text of the present patent application, the expressions "for regulating growth" and "growth regulators" are taken to have their usual meaning, the word "growth" relating to the production of living material and not simply to the modification of the size of plants. "Growth regulators" are therefore to be understood hereafter as meaning products which are capable of modifying the physiology of plants in various ways.

According to the present invention there is provided a method of regulating plant growth (i.e. for modifying the physiology of plants in various ways) at a locus which comprises applying to the locus an effective amount of at least one compound of the general formula I depicted in Figure I:-

$R^3NHC(COOR^1) = CH(COOR^2)$ Figure I

wherein $R^1$ and $R^2$, which are the same, each represent a straight-or branched-chain alkyl group containing from 1 to 6 carbon atoms and $R^3$ represents the phenyl group bearing substituents $R^4$ and $(R^5)_n$ wherein $R^4$ represents the cyano group in the ortho, meta or para position; or represents a straight-or branched-chain alkyl group containing from 1 to 4 carbon atoms, which if fully-substituted by halogen atoms which may be the same or different (for example the trifluoromethyl group), in the metaor para position; or represents the hydroxy group or the fluorine or iodine atom in the meta position; or represents the chlorine or bromine atom, the nitro group or a straight-or branched-chain alkoxy or alkylthio group containing from 1 to 4 carbon atoms, which is fully-substituted by halogen atoms which may be the same or different (for example the trifluoromethoxy or trifluoromethylthio group), in the para position and $R^5$ represents the hydrogen atom and $n$ represents the integer 1;

or $R^4$ represent the fluorine, chlorine, bromine or iodine atom, the cyano group, the hydroxy group, the nitro group or a straight-or branched-chain alkoxy or alkylthio group containing from 1 to 4 carbon atoms, which is fully-substituted by halogen atoms which may be the same or different, or a straight-or branched-chain alkyl group containing from 1 to 4 carbon atoms, which is unsubstituted or fully-substituted by halogen atoms which may be the same or different (preferably the methyl or trifluoromethyl group), and $R^5$ represents the fluorine, chlorine, bromine or iodine atom, the cyano group, the hydroxy group, the nitro group or a straight-or branched-chain alkoxy or alkylthio group containing from 1 to 4 carbon atoms, which is fully-substituted by halogen atoms which may be the same or different, or a straight-or branched-chain alkyl group containing from 1 to 4 carbon atoms, which is unsubstituted or fully-substituted by halogen atoms which may be the same or different - (preferably the methyl or trifluoromethyl group), and $n$ represents the integer 1 or 2; it being understood that when $n$ represents the integer 1 the substituents represented by $R^4$ and $R^5$ do not both represent unsubstituted alkyl groups, hydroxy groups or nitro groups and are different when in the 2,3 or 2,5 positions; and when $n$ represents the integer 2, one of the substituents represented by $R^4$ and $R^5$ is different from the other substituents and does not represent an unsubstituted alkyl group, the positions of the various substituents on the pheny ring referred to above being with respect to the nitrogen atom to which the phenyl ring is attached, preferably in association with a carrier and/or at least one surface-active agent as hereinafter described.

It is to be understood that this specification is intended to embrace the E-form and the Z-form, which arise as a result of geometrical isomerism, and mixtures thereof. The anilinofumarates are preferred. Furthermore, it will be understood by those skilled in the art that the compounds of formula I exhibit tautomerism, and that the forms thus described may be present to a greater or lesser degree and are in a state of dynamic equilibrium with each other as described by S. Toppet et al, Chemistry & Industry, p. 703 (1971). All such forms are embraced by the present invention.

Preferred compounds are those where $n$ is the integer 1 and $R^4$ represents the cyano group, the hydroxy group, the nitro group, the trifluoromethyl group, the trifluoromethoxy group or the fluorine, chlorine, bromine or iodine atom as hereinbefore defined and $R^5$ represents the hydrogen, fluorine chlorine, bromine or iodine atom or the trifluoromethyl group as hereinbefore defined or the methyl group and $R^1$ and $R^2$ are as hereinbefore defined.

Especially preferred compounds are those where $n$ is the integer 1, $R^1$and $R^2$ each represent the methyl or ethyl group, $R^4$ represents the cyano group or the trifluoromethyl group in the meta

position, or the nitro group, the trifluoromethyl group, the trifluoromethoxy group or the chlorine or bromine atom in the para position and $R^5$ represents the hydrogen, fluorine or chlorine atom or the methyl or trifluoromethyl group.

Especially important compounds of general formula I are as follows:-

No. Compound

1 Dimethyl 3,4-dichloroanilinofumarate

2 Diethyl 3,4-dichloroanilinofumarate

3 Dimethyl 4-chloro-3-trifluoromethylanilinofumarate

4 Dimethyl 4-chloro-2-methylanilinofumarate

5 Dimethyl 2,4-dichloroanilinofumarate

6 Dimethyl 3-cyanoanilinofumarate

7 Dimethyl 4-chloroanilinofumarate

8 Diethyl 4-chloroanilinofumarate

9 Dimethyl 4-trifluoromethoxyanilinofumarate

10 Dimethyl 4-bromoanilinofumarate

11 Dimethyl 4-trifluoromethylanilinofumarate

12 Di-isopropyl 3,4-dichloroanilinofumarate

13 Dibutyl 3,4-dichloroanilinofumarate

14 Dipentyl 3,4-dichloroanilinofumarate

15 Dimethyl 3-fluoranilinofumarate

16 Dimethyl 3-iodoanilinofumarate

17 Dimethyl 3-hydroxyanilinofumarate

18 Dimethyl 3-trifluoromethylanilinofumarate

19 Dimethyl 4-cyanoanilinofumarate

20 Dimethyl 4-bromo-3-chloroanilinofumarate

21 Dimethyl 3-chloro-4-fluoroanilinofumarate

22 Dimethyl 3,5-dichloroanilinofumarate

23 Dimethyl 4-nitroanilinofumarate

24 Dimethyl 4-fluoro-3-trifluoromethylanilinofumarate

25 Dimethyl 2-cyanoanilinofumarate

26 Dimethyl 3,5-bistrifluoromethylanilinofumarate

27 Dimethyl 4-chloro-3-cyanoanilinofumarate

The dose of active ingredient applied can vary according to different factors such as, for example, the type of plant or crop to be treated, its stage of development, the climatic conditions and the nature of the land. In practice, the treatments according to the invention are carried out by applying to, for example, a crop-growing area doses of anilinofumarate compound ranging from 0.01 kg/hectare to 20 kg/hectare.

Compound Nos. 1 to 11, 23 and 24 are preferred.

An important method is a method which improves the yield of fruiting crops, especially cereals (e.g. maize, wheat, barley and rice) and brassicae - (e.g. oil seed rape) and more especially legumes - (e.g. soyabean, field bean and dwarf bean), more particularly when applied at a flowering stage as a foliar spray. For this purpose, the compound of the general formula depicted in Figure 1 may suitably be applied at rates of applications of from 0.05 to 8.0 and more especially to 1.0 kg of that compound per hectare.

For example, dimethyl 3,4-dichloroanilinofumarate enhances the yield of dwarf bean (Tests 1 to 3) and soyabean (Test 4) following application as a foliar spray, by increasing the number of pods that are set and enhancing dry matter accumulation. Seed quality may also be improved, for example oil content is increased in oil-producing crops.

A further important method is a method which improves the yield of cereal crops, e.g. winter wheat, when applied at a growth stage which is other than a flowering stage, for example the first node (Zadoks 31) stage or flag leaf extending - (Zadoks 39) stage, as a foliar spray.

An especially important method is a method which improves the yield of legumes grown when sub-optimal conditions (e.g. watered by natural rainfall) prevail, more particularly when applied at or near pollination as a foliar spray.

The method according to the invention for the regulation of plant growth is demonstated by the experiments described in the following tests.

Test 1. Effect of dimethyl 3,4-dichloroanilinofumarate on pod number following application to dwarf bean (var. Blue duet) at the 1st trifoliate leaf expansion stage of growth

| Dose Kg ai/ha | Percentage increase in pod number compared with untreated controls |
|---|---|
| 1.0 | 13 |
| 4.0 | 37 |
| 8.0 | 65 |

The compound was applied using a precision laboratory sprayer fitted with a Teejet 8005E nozzle delivering a spray volume equivalent to 530 l/ha at a pressure of 2.8 kgf/cm². Plants were grown in 9 cm round pots (4 replicate pots/treatment; 1 plant/pot). The treated and unsprayed plants were placed in a greenhouse receiving natural daylight supplemented with mercury lamps providing 7000 lux and heating to give a minimum daytime temperature of 21°C, and given adequate watering.

The spray solutions were prepared by dissolving 0.038g, 0.15g and 0.30 g of test chemical in 20ml acetone. Plants were assessed 28 days after chemical application.

Test 2. Effect of dimethyl 3,4-dichloroanilinofumarate on yield parameters of glasshouse-grown dwarf bean (variety Blue Duet)

| Growth Stage at application | Dose kg ai/ha | Mean height (cm) | Mean total plant fresh weight (g) | Mean number of bean pods | Mean total fresh weight of pods (g) |
|---|---|---|---|---|---|
| Formation of flower | 0.5 | 27 | 48 | 6.0 | 25.2 |
| | 1.0 | 29 | 43 | 6.4 | 21.3 |
| | 2.0 | 26 | 43 | 6.8 | 20.9 |
| | untreated control | 26 | 46 | 5.2 | 21.7 |
| Full flower | 0.5 | 25 | 41 | 4.0 | 21.1 |
| | 2.0 | 30 | 41 | 5.0 | 22.3 |
| | untreated control | 24 | 38 | 3.2 | 17.0 |

Plants were grown in 9cm round pots in John Innes Compost (5 replicate pots/treatment; 1 plant/pot).

The compound was applied using a precision laboratory sprayer fitted with Teejet 8005E nozzle delivering a spray volume equivalent to 530 l/ha at a pressure of $2.8 kgf/cm^2$.

The treated and unsprayed plants were placed in a green-house receiving natural daylight supplemented with mercury lamps providing 7000 lux and heating to give a minimim daytime temperature of 21 °C, and given adequate watering.

The spray solutions were prepared by dissolving 0.016g, 0.032g and 0.064g of dimethyl 3,4-dichloroanilinofumarate in 17ml acetone. Plants were assessed 29 days after chemical application at formation of flower buds and 22 days after application at full flower.

Test 3. Effect of dimethyl 3,4-dichloroanilinofumarate on yield parameters of field-grown dwarf bean (variety Cascade)

| Assessment | Dose kg ai/ha | Mean height (cm) | Mean total plant fresh weight (g) | Mean number of bean pods | Mean total fresh weight of pods (g) |
|---|---|---|---|---|---|
| 28 days after application early (1) maturity | 1.0 | 25 | 97 | 24.4 | 64.8 |
| | untreated control | 21 | 76 | 14.9 | 52.0 |
| 44 days after application full (2) maturity | 1.0 | - | - | 15.6 | 69.9 |
| | untreated control | - | - | 12.0 | 61.9 |

(1) 20 replicate plants assessed
(2) 40 replicate plants assessed

Seeds were hand-sown in prepared ground in seven, 8-metre rows, 50cms apart. The area was netted to prevent bird damage.

The compound was applied at early flowering using a ground crop-sprayer fitted with 2 x 80015 Teejets at 50cm spacing delivering a spray volume of 217 l/ha at a pressure of 2.1 kgf/cm² in low gear.

Two rows were treated with chemical and two rows marked as untreated controls. At early maturity a block of 10 plants were taken from each of the rows and yield parameters determined. A further block of 20 plants from each row was taken at full maturity.

The spray solution was prepared by diluting 5.5ml of dimethyl 3,4-dichloroanilinofumarate formulated as 25% ai w/v emulsifiable concentrate in 300ml water.

Test 4 Effect of dimethyl 3,4-dichloroanilinofumarate on the yield of field-grown soyabean watered by natural rainfall

Experiment 1

| Dose rate kg ai/ha | Mean Number of pods | | Mean Dry weight of pods (g) | |
|---|---|---|---|---|
| | Mid podfill | Maturity | Mid podfill | Maturity |
| 0.56 | 908 | 732 | 144 | 295 |
| 1.12 | 921 | 810 | 119 | 289 |
| 2.24 | 1081 | 854 | 121 | 284 |
| Untreated control | 698 | 590 | 115 | 274 |

Dimethyl 3,4-dichloroanilinofumarate was applied to field-grown soyabean (variety Williams '79) at the early flowering stage to 4.27m x 1.25m plots containing 7 rows 17.8cm apart (5 replicate plots/treatment). Treatments were made with a hand-held boom with backpack sprayer with flat-fan nozzles (Teejet 8002E) delivering a spray volume of 449 litres/ha. Unsprayed plots were kept as controls and the experiments were conducted during the soyabean growing season at Columbus, New Jersey, USA.

At mid podfill and at harvest, plants in the 5 inner rows for 91.4cms along the rows of each plot were hand sampled and the yield components assessed. Dimethyl 3,4-dichloroanilino-fumarate was formulated as a 25% a.i. w/v emulsifiable concentrate and tank-mixed with 0.1% Ortho X-77 surfactant before application.

Experiment 2

| Dose rate kg ai/ha | Mean Number of pods | | Mean Dry weight of pods (g) | |
|---|---|---|---|---|
| | Mid podfill | Maturity | Mid podfill | Maturity |
| 0.14 | 885 | 843 | 218 | 405 |
| 0.28 | 817 | 810 | 176 | 413 |
| 0.56 | 991 | 869 | 215 | 428 |
| 1.12 | 1049 | 983 | 225 | 447 |
| Untreated control | 756 | 631 | 191 | 343 |

Dimethyl 3,4-dichloroanilinofumarate was applied to field-grown soyabean (variety Williams'79) at the early flowering stage to plots (6.1m x 1.83m) containing 6 rows 30.5cms apart (5 replicate plots/treatment). Treatments were made with a hand-held boom with backpack sprayer with flat-fan nozzles (Teejet 8002E) delivering a spray volume of 374 litres/ha. Unsprayed plots were kept as controls and the experiments were conducted during the soyabean growing season at Columbus, New Jersey. USA.

At mid podfill and at harvest plants in an area 4 rows by 91.4cms along the rows, were hand-sampled from each plot and the yield components assessed. Dimethyl 3,4-dichloroanilinofumarate was formulated as a 25% a.i. w/v emulsifiable concentrate and tank mixed with 0.1% Ortho X-77 surfactant before application.

Experiment 3

| Dose rate kg ai/ha | Mean Number of pods | | Mean Dry weight of pods (g) | |
|---|---|---|---|---|
| | Mid podfill | Maturity | Mid podfill | Maturity |
| 0.56 | 1584 | 1116 | 167 | 357 |
| 1.12 | 2045 | 1198 | 213 | 418 |
| Untreated control | 1361 | 842 | 173 | 283 |

Dimethyl 3,4-dichloroanilinofumarate was applied to field-grown soyabean (variety Williams '82) at the early flowering stage. Soyabeans were planted in 30.5cm rows.

Treatments (5 replicate plots/treatment) were made with a hand-held boom and a backpack sprayer with flat-fan nozzles (Teejet 8002E) delivering a spray volume of 374 litres/ha to $1.11m^2$ plots containing 2 rows x 1.83m or 3 rows x 1.22m, selected for uniformity of plant stand. Unsprayed plots were kept as controls and the experiments were conducted during the soyabean growing season at Columbus, New Jersey, U.S.A.

At mid podfill and at harvest all plants were hand-harvested from each plot and the yield components assessed. Dimethyl 3,4-dichloroanilinofumarate was formulated as a 25% a.i. w/v emulsifiable concentrate and tank-mixed with 0.1% Ortho X-77 surfactant before application.

Test 5

| No. | % change in flowering over untreated plants | | | |
|---|---|---|---|---|
| | 0.063 kg/ha | 0.25 kg/ha | 0.5 kg/ha | 1.0 kg/ha |
| 1 | - | +18.2 | +27.3 | +53.3 |
| 2 | - | - | +17.0 | +35.2 |
| 3 | +13.9 | +50.3 | +18.2 | - |
| 4 | +21.3 | +27.6 | +12.8 | - |
| 5 | - | +20.0 | +35.5 | +6.8 |
| 6 | - | +13.6 | +30.3 | +13.6 |
| 7 | - | +20.3 | +25.2 | +24.3 |
| 8 | - | +24.3 | +28.3 | +34.2 |
| 9 | - | +24.0 | +55.7 | - |
| 10 | - | +24.5 | +30.3 | +35.0 |
| 11 | - | +11.4 | +18.3 | +33.9 |
| 12 | - | +23.3 | +11.8 | +27.0 |
| 13 | - | +9.6 | +14.4 | +14.4 |
| 14 | - | +7.2 | +9.6 | +21.6 |
| 15 | - | +15.7 | +9.3 | +3.7 |
| 16 | - | +13.8 | +19.6 | +6.3 |
| 17 | - | +13.8 | +11.6 | +13.8 |
| 18 | - | +2.9 | +13.5 | +17.0 |
| 19 | - | - | +6.4 | +35.2 |
| 20 | - | +20.3 | +23.3 | +27.2 |
| 21 | - | +9.3 | +15.5 | +4.6 |
| 22 | - | +25.0 | +14.9 | +3.7 |

Method

Dwarf french bean variety 'Rondina' seeds were sown in a peat-based compost and raised under glass. Young plants were pricked out into John Innes compost one per 9 cm square pot shortly after emergence and grown on under glasshouse conditions with supplementary lighting as required.

In all tests plants were sprayed at the first trifoliate expanding stage using a laboratory sprayer. All compounds were formulated in acetone and applied to four replicate plants at 500 l/ha and 2.6 kgf/cm$^2$. Plants were returned to glasshouse conditions and were watered twice daily

Assessment of the number of young bean pods, open flowers and swollen flower buds in total were made as the first pods formed, usually two weeks after chemical application. The percentage change in number compared against untreated plants was calculated from the means of these results.

According to a further feature of the present invention, there are provided compositions suitable for regulating plant growth comprising one or more of the aniline derivatives of general formula depicted in Figure I in association with, and preferably homogeneously dispersed in, one or more compatible acceptable diluents or carriers (i.e. diluents or carriers of the type generally accepted in the art as being suitable for use in compositions and which are compatible with compounds of general formula depicted in Figure I). The term "homogeneously dispersed" is used to include compositions in which the compounds of general formula depicted in Figure I are dissolved in the other components. The term "compositions" is used in a broad sense to include not only compositions which are already for use but also concentrates which must be diluted before use. Preferably, the compositions contain from 0.05 to 90% by weight of one or more compounds of general formula depicted in Figure I.

The compositions may contain both a diluent or carrier and surface-active (e.g. wetting, dispering, or emulsifying) agent. Surface-active agents which may be present in compositions of the present invention may be of the ionic or non-ionic types, for example sulphoricinoleates, quaternary ammonium derivatives, products based on condensates of ethylene oxide with alkyl and polyarly phenols, eg. nonyl-or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the compositions according to the present invention may comprise up to 10%, e.g. from 0.05% to 10%, of surface-active agent but, if desired, compositions according to the present invention may comprise higher proportions of surface-active agent, for example up to 15% in liquid emulsifiable suspension concentrates and up to 25% in liquid water soluble concentrates.

Examples of suitable solid diluents or carriers are aluminium silicate, talc, calcined magnesia, kieselguhr, tricalcium phosphate, powered cork, adsorbent carbon black and clays such as kaolin and bentonite. The solid compositions (which may take the form of dusts, granules or wettable powders) are preferably prepared by grinding the compounds of general formula depicted in Figure I with solid diluents or by impregnating the solid diluents or carriers with solutions of the compounds of general formula depicted in Figure I in volatile solvents, evaporating the solvents and, if necessary, grinding the products so as to obtain powders. Granular formulations may be prepared by absorbing the compounds of general formula depicted in Figure I (dissolved in suitable solvents, which may, if desired, be volatile onto the solid diluents or carriers in granular form and, if desired, evaporating the solvents, or by granulating compositions in powder form obtained as described above. Solid compositions, particularly wettable powders and granules, may contain wetting or dispersing agent (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Wettable powders, dispersible granules and liquid compostions in the form of concentrates may be diluted with water or other suitable diluents, for example mineral or vegetable oils, particularly in the case of liquid concentrates in which the diluent or carrier is an oil, to give compositions ready for use. When desired, liquid compositions of the compound of general formula depicted in Figure I may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Preferred compositions according to the present invention are aqueous suspension concentrates which comprise from 10 to 70% of one or more compounds of general formula depicted in Figure I, from 2 to 10% of surface-active agent, from 0.1 to 5% w/v of thickener and from 15 to 87.9% by volume of water; wettable powders which comprise from 10 to 90% w/w of one or more compounds of general formula depicted in Figure I, from 2 to 10% w/w of surface-active agent and from 10 to 88% w/w of solid diluent or carrier; liquid water soluble concentrates which comprise from 5 to 50%, e.g. 10 to 30%, w/v of one or more compounds of general formula depicted in Figure I, from 5 to 25% w/v of surface-active agent and from 25 to 90% e.g. 45 to 85%, by volume of water-miscible solvent, e.g. dimethylformamide, or a mixture of water-miscible solvent and water; liquid emulsifiable suspension concentrates which comprise from 10 to 70% w/v of one or more compounds of general formula depicted in Figure I, 5 to l5% w/v of surface active agent from 0.1 to 5% w/v of thickener and from 10 to 84.9% by volume of organic solvent; granules which comprise from 1 to 90%, e.g. 2 to 10%, w/w of one or more compounds of general formula, depicted in Figure I, from 0.5 to 7%, e.g. 0.5 to 2%, w.w of surface-active agent and from 3 to 98.5%, e.g. 88 to 97.5%, w/w of granular carrier and emulsifiable concentrates which comprise 0.05 to 90% w/v, and preferably from 1 to 60% of one or more compounds of general formula depicted in Figure I, from 0.01 to 10% w/v, and preferably from 1 to 10% w/v, of surface-active agent and from 9.99 to 99.94%, and preferably from 39 to 98.99%, by volume of organic solvent.

Compositions according to the present invention may also comprise the compound of general formula depicted in Figure I in association with, and preferably homogenously dispersed in, one or more pesticidally active compounds and, if desired, one or more compatible pesticidally acceptable diluents or carriers, surface-active agents and conventional adjuvants as hereinbefore described. Examples of pesticidally active compounds which may be included in, or used in conjunction with, the compositions of the present invention, include insecticides, e.g. carbaryl [naphth-1-yl-N-methylcarbamate]; synthetic pyrethroids, e.g. permethrin and cypermethrin; and fungicides, e.g. 2,6-dimethyl-4-tridecyl-morpholine, methyl N-(1-butylcarbamoyl-benzimidazol-2-yl)carbamate, 1,2-bis-(3-methoxycarbonyl-2-thioureido-benzene, isopropyl-1-carbamoyl-3-(3-5-dichlorophenyl)-hydantoin and 1-(4-chloro-phenoxy)-3,3-dimethyl-1-(1,2,4-triazol-1-yl)-butan-2-one. Other biologically active materials which may be included in, or used in conjunction with, the compositions of the present invention are other plant growth regulators, e.g. succinamic acid, (2-chloroethyl)trimethylammonium chloride and 2-chloroethane-phosphonic acid; or fertilizers and seaweed extracts, e.g. containing nitrogen, potassium and phosphorus and trace elements known to be essential to successful plant life, e.g. iron, magnesium, zinc, manganese, cobalt and copper.

Pesticidally active compounds and other biologically active materials which may be included in, or used in conjunction with, the compositions of the present invention, for example those hereinbefore mentioned, and which are acids, may if desired, be utilized in the form of conventional derivatives, for example alkali metal and amine salts and esters.

According to a further feature of the present invention there is provided an article of manufacture comprising at least one of the aniline derivatives of general formula depicted in Figure I or, as is preferred, a composition as hereinbefore described, and preferably a concentrate which must be diluted before use, comprising at least one of the aniline derivatives of general formula depicted in Figure I within a container for the aforesaid derivative or derivatives of general formula depicted in Figure I, or a said composition, and instructions physically associated with the aforesaid container setting out the manner in which the aforesaid derivative or derivatives of general formula depicted in Figure I or composition contained herein is to be used to regulate the growth of plants. The containers will normally be of the types conventionally used for the storage of chemical substances which are solid at normal ambient temperatures and compostions particularly in the form of concentrates, for example cans and drums of metal, which may be internally-lacquered, and plastics materials, bottles of glass and plastic materials and, when the contents of the container is a solid, for example granular plant growth regulator compositions, boxes, for example of cardboard, plastics materials and metal or sacks. The containers will normally be of sufficient capacity to contain amounts of the aniline derivative or compositions sufficient to treat at least one acre of ground to regulate the growth of plants therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to regulate the growth of plants at rates of application between 0.01 kg and 20 kg of active material per hectare in the manner and for the purposes hereinbefore described.

The following Examples illustrate compositions according to the present invention.

EXAMPLE 1

Dimethyl 3,4-dichloroanilinofumarate was formulated as a water soluble concentrate containing

dimethyl 3,4-dichloroanilinofumarate ....... 10% w/v (weight/volume)

Ethylan KEO (nonylphenyl/ethylene oxide condensate containing 9-10 moles of ethylene oxide per mol of phenol ........ 10% w/v

Dimethylformamide ....................to 100% by volume,

by dissolving the Ethylan KEO in a portion of dimethylformamide and then adding the active ingredient with heating and stirring until dissolved. The resulting solution was then made up to 100% by volume by adding the rest of the dimethylformamide.

5 litres of the above formulation may be dissolved in 600 litres of water and sprayed onto 1 hectare of winter wheat at the stage of growth between flag leaf extending and boots visibly swollen.

Dimethyl 3,4-dichloroanilinofumarate may be replaced in the above water soluble concentrate by any other compound of the general formula depicted in Figure I.

EXAMPLE 2

A wettable powder was formed from:-

| | |
|---|---|
| Dimethyl 3,4-dichloroanilinofumarate ....... | 50% w/w (weight/weight) |
| Nekal BX (sodium salt of alkyl naphthalene sulphonate) .................... | 10% w/w |
| Sodium lignosulphonate .................... | 3% w/w |
| Sopropon T36 (sodium salt of polycarboxylic acid) ....................... | 0.5% w/w |
| Silica filler .............................. | to 100% w/w |

by mixing the ingredients and grinding the mixture in a hammer-mill to give a wettable powder which may be diluted with water and applied at an application rate of 2 kg of wettable powder in 300 litres of spray fluid per hectare in a crop of soyabeans at the flowering stage.

Similar wettable powders may be prepared as described above by replacing the dimethyl 3,4-dichloroanilinofumarate by other compounds of the general formula depicted in Figure I.

EXAMPLE 3

An aqueous suspension concentrate was formed from:-

| | |
|---|---|
| Dimethyl 3,4-dichloroanilinofumarate ....... | 50% w/v |
| Ethylan BCP ............................... | 1.0% w/v |
| Sopropon T36 (sodium salt of polycarboxylic acid) ..................... | 0.2% w/v |
| Ethylene glycol ............................ | 5% w/v |
| Rhodigel 23 (polysaccharide xanthan gum thickener) ........................... | 0.15% w/v |
| distilled water ..................... | to 100% by volume |

by intimately mixing the ingredients and grinding in a ball-mill for 24 hours. The concentrate thus obtained may be dispersed in water and applied at an application rate of 1 kg of aqueous suspension concentrate in 300 litres of spray fluid per hectare in a crop of winter barley at the stage of growth between flag leaf extending and boots visibly swollen.

Similar aqueous suspension concentrates may be prepared as described above by replacing the dimethyl 3,4-dichloroanilinofumarate by other compounds of the general formula depicted in Figure I.

EXAMPLE 4

An emulsifiable suspension concentrate was formed from:-

| | |
|---|---|
| Dimethyl 3,4-dichloroanilinofumarate ....... | 50% w/v |
| Ethylan TU (a nonyl phenol/ethylene oxide condensate containing 10 moles of ethylene oxide per mol of phenol) ....... | 10% w/v |
| Bentone 38 (an organic derivative of special magnesium montmorillonite thickener) ................................ | 0.5% w/v |
| Aromasol H (an aromatic solvent consisting predominantly of isomeric trimethylbenzenes) ................... | to 100% by volume |

by intimately mixing the ingredients and grinding in a ball-mill for 24 hours. The emulsifiable suspension concentrate thus obtained may be diluted with water and applied at an application rate of 1.5 kg of emulsifiable suspension concentrate in 100 litres of spray fluid per hectare in an emerged crop of winter wheat.

Similar emulsifiable suspension concentrates may be prepared as described above by replacing the dimethyl 3,4-dichloroanilinofumarate by other compounds of the general formula depicted in Figure I.

EXAMPLE 5

An emulsifiable concentrate was formed from:

| | |
|---|---|
| Dimethyl 3,4-dichloroanilinofumarate ....... | 25% w/v |
| Ethylan C40 AH (polyethylene glycol unsaturated fatty acid ester)/ Arylan CA (70% solution of calcium dodecyl benzenesulphonate) ................. | 7.5% w/v |
| Cyclohexanone .............................. | 25% v/v |
| Solvesso 150 (aromatic C10 fraction of petroleum) ........................ | to 100% by volume |

The emulsifiable concentrate thus obtained may be diluted with water and applied at an application rate of 2 -4 litres of emulsifiable concentrate in 300 litres of spray fluid per hectare in a crop of soyabean at an early flowering stage of growth.

Similar emulsifiable concentrates may be prepared by replacing the dimethyl 3,4-dichloroanilinofumarate by other compounds of the general formula depicted in Figure I.

EXAMPLE 6

Granules were formed from:-

| | |
|---|---|
| Dimethyl 3,4-dichloroanilinofumarate ....... | 5% w/w |
| Ethylan BCP .............................. | 1% w/w |
| Oleic acid ................................ | 1% w/w |
| Aromasol H ................................ | 12% w/w |
| 30/60 Attapulgite granules (sorptive silica clay) .................... | 81% w/w |

by mixing the anilinofumarate, Ethylan BCP, oleic acid and Aromasol H and spraying the mixture onto the Attapulgite granules. The granules thus obtained may be applied at an application rate of 20 kg of granules per hectare in a crop of soyabean at the flowering stage.

Similar granules may be prepared as described above by replacing the dimethyl 3,4-dichloroanilinofumarate by other compounds of the general formula depicted in Figure I.

EXAMPLE 7

A water soluble concentrate was formed from:-

| | |
|---|---|
| Dimethyl 3,4-dichloroanilinofumarate ....... | 10% w/v |
| Ethylan KEO ................................ | 10% w/v |
| Dimethylformamide .................... | to 100% by volume |

by dissolving the Ethylan KEO in a portion of dimethylformamide and then adding the anilinofumarate derivative with heating and stirring until dissolved. The resulting solution was then made up to 100% by volume with the dimethylformamide by adding the rest of the dimethylformamide. The water soluble concentrate thus obtained may be diluted with water and applied at an application rate of 10 litres of water soluble concentrate in 1200 to 2000 litres of spray fluid per hectare in a crop of winter wheat at the stage of growth between flag leaf extending and boots visibly swollen.

Similar water solube concentrates may be prepared as described above by replacing the dimethyl 3,4-dichloroanilinofumarate by other compounds of the general formula depicted in Figure I.

EXAMPLE 8

A wettable powder was formed from:-

| | |
|---|---|
| Dimethyl 3,4-dichloroanilinofumarate ....... | 90% w/w |
| Arylan S (sodium dodecyl benzene sulphonate) .............................. | 2% w/w |
| Darvan No. 2 (sodium lignosulphate) ........ | 5% w/w |
| Celite PF ................................ | 3% w/w |

by mixing the ingredients and grinding the mixture in a hammer-mill to give a wettable powder which may be diluted with water and applied at an application rate of 1 kg of wettable powder in 300 litres of spray fluid per hectare in an emerged crop of winter wheat.

Similar wettable powders may be prepared as described above by replacing the dimethyl 3,4-dichloroanilinofumarate by other compounds of the general formula depicted in Figure I.

EXAMPLE 9

Water-dispersible granules were formed from:-

| | |
|---|---|
| Dimethyl 3,4-dichloroanilinofumarate ....... | 90% w/w |
| Arylan S (sodium dodecyl benzene sulphonate) ............................... | 2% w/w |
| Darvan No. 2 (sodium lignosulphonate) ...... | 5% w/w |
| Celite PF .................................. | 3% w/w |

by mixing the ingredients and grinding in a hammer-mill to give a wettable powder, which was then throroughly mixed with sufficient water (up to 5% w/w) to give a 'dough'. The 'dough' thus obtained was granulated by passing through an extruder and the granules were dried to remove water. The water-dispersible granules thus obtained may be diluted with water and applied at an application rate of 1 kg of granules in 300 litres of spray fluid per hectare in a crop of maize during pollination. Similar water dispersible granules may be prepared as described above by replacing the dimethyl 3,4-dichloroanilinofumarate by other compounds of the general formula depicted in Figure I.

The compounds of the general formula depicted in Figure I can be prepared by the application or adaptation of known methods (i.e. methods heretofore used or described in the chemical literature), for example, by the reaction of a substituted aniline of the general formula :-$R^3NH_2$ II,

wherein $R^3$ is as hereinbefore defined with the corresponding dialkyl acetylendicarboxylate of the general formula:-$R^1OOCC{\equiv}CCOOR^2$ III,

wherein $R^1$ and $R^2$ are as hereinbefore defined or the corresponding dialkyl oxalacetate of the general formula:-$R^1OOCC(=O)CH_2COOR^2$ IV,

wherein $R^1$ and $R^2$ are as hereinbefore defined, or its mono-alkali metal salt, e.g. its mono-sodium salt, optionally followed by isomerisation by methods known per se.

The reaction of the substituted aniline of general formula II with a dialkyl acetylenedicarboxylate of general formula III may be effected in the corresponding alkanol or in the presence of an inert organic solvent, e.g. diethyl ether or benzene, at a temperature from ambient temperature to the reflux temperature of the reaction mixture, optionally in the presence of a Lewis acid catalyst.

The reaction of the substituted aniline of general formula II with a dialkyl oxalacetate of general formula IV (or its mono-alkali metal salt) may be effected in the presence of an inert organic solvent e.g. glacial acetic acid or benzene, at a temperature from ambient temperature to the reflux temperature of the reaction mixture, optionally separating off water as it is produced.

The compounds of general formula I

wherein $R^1$ and $R^2$, which are the same, each represent a straight-or branched-chain alkyl group containing from 1 to 6 carbon atoms and $R^3$ represents a phenyl group bearing substituents $R^4$ and $R^5)_n$:

wherein $R^4$ represents a cyano group; a straight-or branched-chain alkyl group containing from 1 to 4 carbon atoms which is fully substituted by halogen atoms, which may be the same or different, in the meta or para position; a hydroxy group or a fluorine or iodine atom in the metaposition; a chlorine or bromine atom or a nitro group or straight-or branched-chain alkoxy or alkylthio group containing from 1 to 4 carbon atoms which is fully substituted by halogen atoms, which may be the same or different, in the para position and $R^5$ represents a hydrogen atom and n is 1;

or $R^4$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms

which is unsubstituted or fully substituted by halogen atoms, which may be the same or different, and $R^5$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halolgen atoms, which may be the same or different, and n is 1 or 2;

it being understood that:

a) if n is 1, $R^4$ and $R^5$ do not both represent unsubstituted alkyl groups, hydroxy groups or nitro groups and are different when in the 2,3-or 2,5-positions,

b) if n is 2, one of the substituents represented by $R^4$ and $R^5$ is different from the others and does not represent an unsubstituted alkyl group,

c) if $R^1$ and $R^2$ both represent methyl groups.

$R^3$ does not represent a

4-chlorophenyl, 2-cyanophenyl, 3-hydroxyphenyl,

3-trifluoromethylphenyl, 4-nitrophenyl, 4-chloro-2-cyanophenyl,

4-bromo-2-cyanophenyl, 2-chloro-5-trifluoromethylphenyl,

3,5-dichlorophenyl, 2-nitro-3,4-dimethylphenyl,

2-nitro-4-chlorophenyl, 2-nitro-4-methylphenyl, 2-methyl-

3-nitrophenyl, 5-nitro-2-methylphenyl, 4-nitro-2-methylphenyl or

2-chloro-5-nitrophenyl group.

d) if $R^1$ and $R^2$ both represent ethyl groups

$R^3$ does not represent a

4-chlorophenyl, 4-bromophenyl, 2,4-dichlorophenyl or

3,4-dichlorophenyl group, the positions of the various substituents on the phenyl ring referred to above being with respect to the nitrogen atom to which the phenyl ring is attached, are new and form a feature of the present invention.

According to a further feature of the present invention there is provided a process as hereinbefore described for the preparation of the compounds of general formula I which are new.

By the term "methods known per se" as used in this specification is meant methods heretofore used or described in the literature.

The following Examples illustrate the preparation of the compounds of the general formula depicted in Figure I.

Chromatography, where indicated, was carried out using a silica column (Merck 230-400 mesh : 25 lb sq in $^{-1}$ (1.76 Pa)) with the eluent hereinafter stated.

EXAMPLE 10

A solution of dimethyl acetylendicarboxylate - (200g) in anhydrous methanol (750 ml) was added in a single addition to a stirred solution of 3,4-dichloroaniline (228.1g) in anhydrous methanol - (750ml). An exothermic reaction occurred causing the internal temperature to rise to 75°C. The stirring was discontinued when this reaction had subsided (after 45 minutes) and the reaction mixture was allowed to cool to laboratory temperature. Most of the methanol was then removed from the reaction mixture by evaporation under reduced pressure and the resultant slurry was filtered to give a green residue. This solid residue was washed with ice-cold methanol (200 ml) and dried in a desiccator to give dimethyl 3,4-dichloroanilino fumarate (324.8g), m.p. 72.5 -74.0°C, in the form of a yellow-green powder.

Dimethyl acetylenedicarboxylate and 3,4-dichloroaniline are known compounds.

EXAMPLE 11

Compounds 3, 4, 5, 6, 9, 10, 11, 15, 16, 19, 20 and 21

By proceeding in a similar manner to Example 10, but replacing the 3,4-dichloroaniline by the hereinafter indicated appropriately substituted aniline, there were obtained:

Dimethyl 4-chloro-3-trifluoromethylanilinofumarate in the form of a yellow oil, following chromatography using a mixture of diethyl ether and hexane (1:2), from 4-chloro-3-trifluoromethylaniline.

Dimethyl 4-chloro-2-methylanilinofumarate, m.p. 68-69.5°C, in the form of a yellow solid, following chromatography using a mixture of diethyl ether and hexane (1:2), from 4-chloro-2-methylaniline.

Dimethyl 2,4-dichloroanilinofumarate, m.p. 100-101°C, in the form of fawn coloured cystals, after crystallisation from methanol, from 2,4-dichloroaniline.

Dimethyl 3-cyanoanilinofumarate, m.p. 72.5-73.5°C, in the form of a yellow-green solid, after crystallisation from methanol, from 3-cyanoaniline.

Dimethyl 4-trifluoromethoxyanilinofumarate, m.p. 43-46°C, in the form of a yellow powder, after crystallisation from hexane, from 4-trifluoromethoxyaniline.

Dimethyl 4-bromoanilinofumarate, m.p. 86-87°C, in the form of pale yellow crystals, after crystallisation from methanol, from 4-bromoaniline.

Dimethyl 4-trifluoromethylanilinofumarate, in the form of a yellow oil, following chromatography using dichloromethane, from 4-trifluoromethylaniline.

Dimethyl 3-fluoroanilinofumarate, in the form of a yellow oil, following chromatography using a mixture of diethyl ether and hexane (1:1), from 3-fluoroaniline.

Dimethyl 3-iodoanilinofumarate, in the form of a yellow oil, following chromatography using a mixture of diethyl ether and hexane (1:1), from 3-iodoaniline.

Dimethyl 4-cyanoanilinofumarate, m.p. 109-111°C, in the form of colourless crystals, after crystallisation from methanol, from 4-cyanoaniline.

Dimethyl 4-bromo-3-chloroanilinofumarate, m.p. 100-101°C, in the form of a pale yellow solid, from 4-bromo-3-chloroaniline.

Dimethyl 3-chloro-4-fluoroanilinofumarate, in the form of an orange oil, following chromatography using a mixture of diethyl ether and hexane (1:2), from 3-chloro-4-fluoroaniline.

EXAMPLE 12

Compounds 2 and 12

By proceeding in a similar manner to Example 10, but replacing the dimethyl acetylenedicarboxylate by the hereinafter indicated appropriately substituted dialkyl acetylenedicarboxylate, there were obtained:

Diethyl 3,4-dichloroanilinofumarate, m.p. 65.5-66°C, in the form of a green solid, after crystallisation from ethanol, from diethyl acetylenedicarboxylate.

Di-isopropyl 3,4-dichloroanilinofumarate, in the form of a yellow oil, following chromatography using a mixture of diethyl ether and hexane (1:2), from di-isopropyl acetylenedicarboxylate.

EXAMPLE 13

Compounds 13 and 14

By proceeding in a similar manner to Example 10, but replacing the dimethyl acetylenedicarboxylate by the hereinafter indicated appropriately substituted dialkyl acetylenedicarboxylate, and carrying out the reaction in chloroform in place of methanol at the reflux temperature of the reaction, there were obtained:

Dibutyl 3,4-dichloroanilinofumarate, in the form of a yellow oil, following chromatography using a mixture of diethyl ether and hexane (1:3), from dibutyl acetylenedicarboxylate.

Dipentyl 3,4-dichloroanilinofumarate, in the form of a yellow oil, following chromatography using a mixture of diethyl ether and hexane (1:5), from dipentyl acetylenedicarboxylate.

EXAMPLE 14

Compound 2

A mixture of ethyl oxalacetate mono sodium salt (5 g) and 3,4-dichloroaniline (6 g) in glacial acetic acid (17.5 ml) was stirred at 40°C for 4 hours. The resulting brown solution was then kept at laboratory temperature for 16 hours, poured onto ice (65 g) and basified with aqueous sodium hydroxide solution (50% w/v). The basified reaction mixture was extracted with dichloromethane and the organic layer was washed successively with hydrochloric acid (2N) and aqueous sodium hydroxide solution (2N) and dried over anhydrous magnesium sulphate. Evaporation of the solvent under diminished pressure gave a green-brown oil

which crystallised on standing. This was recrystallised from ethanol to give diethyl 3,4-dichloroanilinofumarate (2.3 g), m.p. 65-65.5°C, in the form of green crystals.

With authentic diethyl 3,4-dichloroanilinofumarate prepared from diethyl acetylenedicarboxylate, mixed m.p. 65-65.5°C.

EXAMPLE 15

Compounds 24, 26 and 27

By proceeding in a similar manner to Example 10, but replacing the 3,4-dichloroaniline by the hereinafter indicated appropriately substituted aniline, there were obtained:

Dimethyl 4-fluoro-3-trifluoromethylanilinofumarate in the form of a yellow oil, following chromatography using dichloromethane, from 4-fluoro-3-trifluoromethylaniline.

Dimethyl 3,5-bistrifluoromethylanilinofumarate in the form of a yellow oil, following chromatography using a mixture of diethyl ether and hexane - (3:17), from 3,5-bistrifluoromethylaniline.

Dimethyl 4-chloro-3-cyanoanilinofumarate, m.p. 112.5-113.5°C, in the form of an off-white solid, from 4-chloro-3-cyanoaniline.

Known compounds of general formula I are as follows:-

Dimethyl 4-chloroanilinofumarate

Dimethyl 2-cyanoanilinofumarate

Dimethyl 4-chloro-2-cyanoanilinofumarate

Dimethyl 4-bromo-2-cyanoanilinofumarate

Dimethyl 2-chloro-5-trifluoromethylanilinofumarate

Dimethyl 3,5-dichloroanilinofumarate

Dimethyl 3-hydroxyanilinofumarate

Dimethyl 3-trifluoromethylanilinofumarate

Dimethyl 4-nitroanilinofumarate

Dimethyl 2-nitro-3,4-dimethylanilinofumarate

Dimethyl 2-nitro-4-chloroanilinofumarate

Dimethyl 2-nitro-4-methylanilinofumarate

Dimethyl 2-methyl-3-nitroanilinofumarate

Dimethyl 5-nitro-2-methylanilinofumarate

Dimethyl 4-nitro-2-methylanilinofumarate

Dimethyl 2-chloro-5-nitroanilinofumarate

Diethyl 4-chloroanilinofumarate

Diethyl 4-bromoanilinofumarate

Diethyl 2,4-dichloroanilinofumarate

Diethyl 3,4-dichloroanilinofumarate

and their geometrically isomeric and tautomeric forms.

No plant growth regulating activity nor herbicidal activity was previously disclosed for these known compounds of general formula I.

## Claims

1. A method of regulating plant growth at a locus which comprises applying to the locus an effective amount of a compound of the general formula:

$R^3NHC(COOR^1) = CH(COOR^2)$ (I)

wherein $R^1$ and $R^2$, which are the same, each represent a straight-or branched-chain alkyl group containing from 1 to 6 carbon atoms and $R^3$ represents a phenyl group bearing substituents $R^4$ and -$(R^5)_n$ ;

wherein $R^4$ represents a cyano group, a straight-or branched-chain alkyl group containing from 1 to 4 carbon atoms which is fully substituted by halogen atoms, which may be the same or different, in the meta or para position; a hydroxy group or a fluorine or iodine atom in the meta position; a chlorine or bromine atom or a nitro group or straight-or branched-chain alkoxy or alkylthio group containing from 1 to 4 carbon atoms which is fully substituted by halogen atoms, which may be the same or different, in the para position and $R^5$ represents a hydrogen atom and n is 1;

or $R^4$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halogen atoms, which may be the same or different, and $R^5$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halogen atoms, which may be the same or different, and n is 1 or 2;

it being understood that:

a) when n is 1, $R^4$ and $R^5$ do not both represent unsubstituted alkyl groups, hydroxy groups or nitro groups and are different when in the 2,3-or 2,5-positions,

b) when n is 2, one of the substituents represented by $R^4$ and $R^5$ is different from the others and does not represent an unsubstituted alkyl group,

the positions of the various substituents on the phenyl ring referred to above being with respect to the nitrogen atom to which the phenyl ring is attached.

2. A method according to claim 1 wherein, in the compound of formula (I), $R^4$ represents a cyano, hydroxy, nitro, trifluoromethyl or trifluoromethoxy group or a fluorine, chlorine, bromine or iodine atom, $R^5$ represents a hydrogen, fluorine, chlorine, bromine or iodine atom or a trifluoromethyl or methyl group and n is 1.

3. A method according to claim 1 or 2 wherein the compound of formula (I) is an anilinofumarate.

4. A method according to any one of claims 1 to 3 which comprises applying the compound of formula (I) to the locus of fruiting crops at a flowering stage as a foliar spray.

5. A method according to any one of claims 1 to 3 which comprises applying the compound of formula (I) to the locus of cereal crops at a growth stage which is other than a flowering stage as a foliar spray.

6. A method according to any one of claims 1 to 3 which comprises applying the compound of formula (I) to the locus of legumes at or near pollination as a foliar spray.

7. A plant growth modifying composition comprising an active compound of the general formula:

$R^3NHC(COOR^1) = CH(COOR^2)$ (I)

wherein $R^1$, $R^2$ and $R^3$ are as defined in claim 1 in association with an agriculturally acceptable carrier or diluent.

8. A composition according to claim 7 which additionally comprises a surface-active agent.

9. A compound of the general formula:

$R^3NHC(COOR^1) = CH(COOR^2)$ (I)

wherein $R^1$ and $R^2$, which are the same, each represent a straight-or branched-chain alkyl group containing from 1 to 6 carbon atoms and $R^3$ represents a phenyl group bearing substituents $R^4$ and -$(R^5)_n$:

wherein $R^4$ represents a cyano group; a straight-or branched-chain alkyl group containing from 1 to 4 carbon atoms which is fully substituted by halogen atoms, which may be the same or different, in the <u>meta</u> or <u>para</u> position; a hydroxy group or a fluorine or iodine atom in the <u>meta</u> position; a chlorine or bromine atom or a nitro group or straight-or branched-chain alkoxy or alkylthio group containing from 1 to 4 carbon atoms which is fully substituted by halogen atoms, which may be the same or different, in the <u>para</u> position and $R^5$ represents a hydrogen atom and n is 1;

or $R^4$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halogen atoms, which may be the same or different, and $R^5$ represents a fluorine, chlorine, bromine or iodine atom, a cyano, hydroxy or nitro group, or a straight-or branched-chain alkoxy or alkylthio group containing from 1 to 4 carbon atoms which is fully substituted by halogen atoms, which may be the same or different, or a straight-or branched-chain alkyl group containing from 1 to 4 carbon atoms which is unsubstituted or fully substituted by halogen atoms, which may be the same or different, and n is 1 or 2;

it being understood that:

a) if n is 1, $R^4$ and $R^5$ do not both represent unsubstituted alkyl groups, hydroxy groups or nitro groups and are different when in the 2,3-or 2,5-positions,

b) if n is 2, one of the substituents represented by $R^4$ and $R^5$ is different from the others and does not represent an unsubstituted alkyl group,

c) if $R^1$ and $R^2$ both represent methyl groups $R^3$ does not represent a 4-chlorophenyl, 2-cyanophenyl, 3-hydroxyphenyl, 3-trifluoromethylphenyl, 4-nitrophenyl, 4-chloro-2-cyanophenyl, 4-bromo-2-cyanophenyl, 2-chloro-5-trifluoromethylphenyl, 3,5-dichlorophenyl, 2-nitro-3,4-dimethylphenyl, 2-nitro-4-chlorophenyl, 2-nitro-4-methylphenyl, 2-methyl-3-nitrophenyl, 5-nitro-2-methylphenyl, 4-nitro-2-methylphenyl or 2-chloro-5-nitrophenyl group,

d) if $R^1$ and $R^2$ both represent ethyl groups $R^3$ does not represent a 4-chlorophenyl, 4-bromophenyl, 2,4-dichlorophenyl or 3,4-dichlorophenyl group, the positions of the various substituents on the phenyl ring referred to above being with respect to the nitrogen atom to which the phenyl ring is attached.

10. A process for the preparation of a compound according to claim 9 which comprises reacting a substituted aniline of the general formula:-

$R^3NH_2$ II,

wherein $R^3$ is as defined in claim 9 with the corresponding dialkyl acetylenedicarboxylate of the general formula:

$R^1OOCC{\equiv}CCOOR^2$ III,

wherein $R^1$ and $R^2$ are as defined in claim 9 or the corresponding dialkyl oxalacetate of the general formula:

$R^1OOCC(=O)CH_2COOR^2$ IV,

wherein $R^1$ and $R^2$ are as defined in claim 9, or its mono-alkali metal salt, optionally followed by isomerisation by methods known per se.